# EUROPEAN PATENT APPLICATION

(11) **EP 1 757 687 A1**
(43) Date of publication of application: **28.02.2007**
(21) Application number: 05730475.0
(22) Date of filing: 14.04.2005
(51) Int. Cl.: C12N 15/09, C07K 7/06, C12N 15/11

(54) **HLA-BINDING PEPTIDE, PRECURSOR THEREOF, DNA FRAGMENT ENCODING THE SAME AND RECOMBINANT VECTOR**

(30) Priority: 30.04.2004 JP 2004135652; 17.09.2004 JP 2004272314; 25.02.2005 JP 2005050164
(71) Applicant: NEC CORPORATION, Tokyo (JP); Kochi University, Kochi-shi Kochi 7808520 (JP)
(72) Inventor: MIYAKAWA, Tomoya, c/o NEC Corporation, Tokyo, 1088001 (JP); UDAKA, Keiko, 7838505 (JP)
(74) Representative: Vossius & Partner
(86) International application number: PCT/JP2005/007231
(87) International publication number: WO 2005/105993

(57) **Abstract**

An HLA-binding peptide binding to an HLA-A type molecule, said HLA-binding peptide comprising at least one type of amino acid sequence selected from the group consisting of SEQ ID NOS: 1 to 183, and not less than 8 and not more than 11 amino acid residues is provided. Any of the amino acid sequence is predicted to have the binding property to a human HLA-A type molecule by a predicting program using an active learning experiment method as illustrated in FIG. 1.

## Description

### TECHNICAL FIELD

The present invention relates to HLA-binding peptides, precursor thereof and DNA fragments and recombinant vectors coding for those sequences.

### BACKGROUND ART

When one is infected with a virus such as hepatitis C virus (HCV) a virus specific immune response is induced to eliminate the virus following a defense by the innate immune system.

When a specific immune response is induced isolated viral particles lose their infectivity by neutralizing antibodies and are subsequently eliminated. In the other words, virus infected cells are lysed by cytotoxic T lymphocytes (CTLs). CTL recognizes as antigen an epitope peptide presented by an HLA class I molecule. Such epitope peptides are 8 to 11 amino acids in length. Therefore, it is critical to identify viral epitope peptides in order to develop a therapeutic vaccine against the virus.

A technique of this kind is known from Patent Publication 1. Patent Publication 1 states that an oligopeptide formed from a specific amino acid sequence has the property of binding to an HLA.
[Patent Publication 1] Japanese Patent Application Laid-open No. H8-151396 (1996)

### DISCLOSURE OF THE INVENTION

However, the conventional technique described in the above-mentioned publication has room for improvement on the following points.

Firstly, it is unclear whether or not the HLA-binding peptide of the above-mentioned publication binds to an HLA molecule effectively, and there is still room for improvement in terms of the property of binding to an HLA.

Secondly, it is stated that the HLA-binding peptide of the above-mentioned publication has the property of binding to HLA-DQ4. However, it is unclear whether or not it binds to an HLA-A2 type molecule (product of the HLA-A*0201 gene and the like), which is often seen in European and American people, and an HLA-A24 type molecule (product of the HLA-A*2402 gene and the like), which is often seen in Japanese people.

The present invention has been accomplished under the above-mentioned circumstances, and provides HLA-binding peptides that exhibit high-affinity binding to a specific type of HLA molecule.

According to the present invention, there is provided an HLA-binding peptide binding to an HLA-A type molecule, the HLA-binding peptide containing at least one type of amino acid sequence selected from the group consisting of SEQ ID NOS: 1 to 183, and consisting of not less than 8 and not more than 11 amino acid residues.

Furthermore, according to the present invention, there is provided the HLA-binding peptide comprising at least one type of amino acid sequence selected from the group consisting of SEQ ID NOS: 1, 2, 3, 5, 8, 12, 13, 14, 16, 17, 18, 19, 22, 23, 25, 27, 34, 37, 38, 40, 42, 45, 48, 49, 52, 53, 54, 55, 56, 58, 59, 60, 61, 62, 63, 64, 65, 67, 71, 72, 74, 75, 76, 84, 86, 87, 90, 91, 92, 93, 94, 96, 97, 98, 100, 101, 102, 104, 106, 107, 108, 109, 110, 112, 123, 124, 126, 127, 131, 132, 133, 134, 135, 136, 137, 139, 141, 142, 146, 147, 149, 150, 152, 162, 170, 173, 176, 177, and 179.

Moreover, according to the present invention, there is provided an HLA-binding peptide binding to an HLA-A type molecule, the HLA-binding peptide containing an amino acid sequence formed by deletion, substitution, or addition of one or two amino acid residues of the amino acid sequence contained in the above-mentioned HLA-binding peptide, and consisting of not less than 8 and not more than 11 amino acid residues.

In this way, the construct containing an amino acid sequence formed by deletion, substitution, or addition of one or a few amino acid residues of a specific amino acid sequence that has the property of binding to an HLA-A type molecule can also exhibit the similar effect to that of the above-mentioned HLA-binding peptide.

Furthermore, according to the present invention, there is provided a DNA segment containing a DNA sequence coding for the above-mentioned HLA-binding peptide.

Moreover, according to the present invention, there is provided a recombinant vector containing a DNA sequence coding for the above-mentioned HLA-binding peptide.

Furthermore, according to the present invention, there is provided an HLA-binding peptide precursor changing within a mammalian body into the above-mentioned HLA-binding peptide.

In accordance with the present invention, since it includes a specific amino acid sequence, an HLA-binding peptide that has excellent properties in binding to an HLA-A type molecule can be obtained.

### BRIEF DESCRIPTION OF THE DRAWINGS

The above-mentioned object, other objects, features, and advantages will become more apparent from preferred embodiments explained below by reference to the attached drawing.

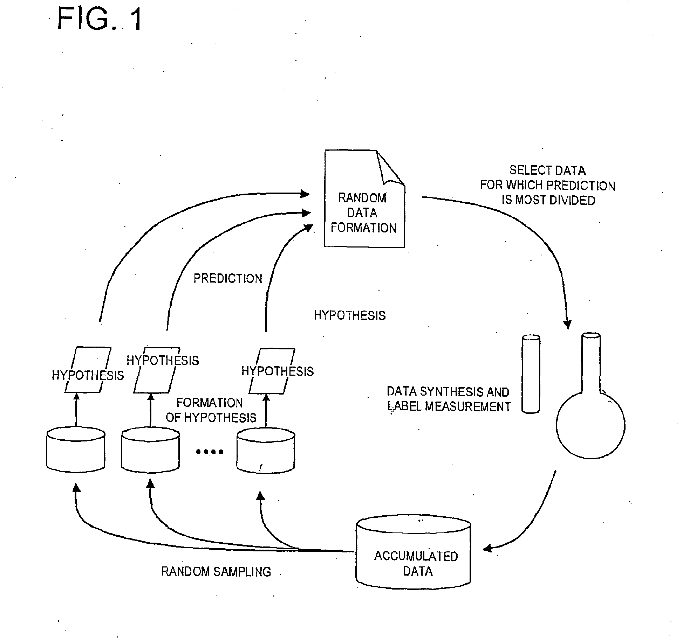

[FIG. 1] A schematic drawing for explaining an active learning experiment design used in an embodiment.

### BEST MODE FOR CARRYING OUT THE INVENTION

Modes for carrying out the present invention are explained below by reference to a drawing. In all the drawings, the same constitutional elements are denoted by the same reference numerals and symbols, so that the explanation will not be repeated.

### <Embodiment 1>

In this embodiment a peptide that contains an amino acid sequence for which the binding to an HLA molecule, predicted by a hypothesis obtained using an active learning experiment method (Japanese Patent Application Laid-open No. H11-316754 (1999)), is 3 or greater in terms of a -log Kd value, and consists of not less than 8 and not more than 11 amino acid residues is used as a candidate for an HLA-binding peptide. As a result of a binding experiment, it has been confirmed that these peptides are actually HLA-binding peptides.

As a result, a large number of HLA-binding peptides that have excellent properties in binding to an HLA-A type molecule because they contain an amino acid sequence for which the binding to the HLA molecule in terms of a - log Kd value is 3 or greater could be obtained efficiently.

Specifically, the HLA-binding peptide related to this embodiment is an HLA-binding peptide that binds to an HLA-A type molecule, and that contains at least one type of amino acid sequence selected from the group consisting of SEQ ID NOS: 1 to 183, which will be described later, and that consists of not less than 8 and not more than 11 amino acid residues.

Among human HLA-A types, about 50% of Japanese people have the HLA-A24 type. For European and American people such as German people have the HLA-A2 type.

All of these sequences herein mentioned are sequences consisting of 9 amino acid residues contained in a certain genome protein of HCV (hepatitis C virus).

The sequences of SEQ ID NOS: 1 to 44 are given in Table 1 below.

**TABLE. 1 HLA - A24 - BINDING PEPTIDE**

| SEQ ID NO | D90208 PREDICTED SCORE | PREDICTED SCORE | SEQ NAME | BINDING EXPERIMENT DATA |
|---|---|---|---|---|
| 1 | ILPCSFTTL | 6.9039 | 674 | 7.6571 |
| 2 | VILDSFDPI | 6.293 | 2251 | 5.32417 |
| 3 | RYAPVCKPL | 6.2755 | 2132 | 6,14848 |
| 4 | FWAKHMWNF | 6.0822 | 1760 | |
| 5 | ALYDVVSTL | 6.0484 | 2593 | 6.38942 |
| 6 | TVLSDFKTW | 6.0021 | 1986 | |
| 7 | PYIEQGMQL | 5.9628 | 1716 | |
| 8 | WHYPCTVNF | 5.921 | 616 | 6.38729 |
| 9 | KFPPALPIW | 5.8662 | 2280 | |
| 10 | TYSTYCKFL | 5.8658 | 1292 | |
| 11 | AYSQQTRGL | 5.831 | 1031 | |
| 12 | AQPGYPWPL | 5.8258 | 77 | 5.36419 |
| 13 | ILMTHFFSI | 5.8071 | 2843 | 7.89519 |
| 14 | SYTWTGALI | 5.8059 | 2422 | 7.12954 |
| 15 | SPPAVPQIF | 5.7982 | 1215 | |
| 16 | LLPRRGPRL | 5.7503 | 36 | 7.71195 |
| 17 | ALYGVWPLL | 5.7447 | 789 | 6.98038 |
| 18 | LMTHFFSIL | 5.7443 | 2844 | 5.9169 |
| 19 | LLKRLHQWI | 5.7425 | 1956 | 6.857254 |
| 20 | YILLLFLLL | 5.738 | 718 | |
| 21 | ARPDYNPPL | 5.7226 | 2289 | |
| 22 | AYYSMVGNW | 5.7076 | 360 | 6.46991 |
| 23 | FLARLIWWL | 5.6847 | 838 | 6.17696 |
| 24 | SQLDLSGWF | 5,6728 | 2962 | |
| 25 | SMLTDPSHI | 5.6657 | 2173 | 6.94013 |
| 26 | EYILLLFLL | 5.6643 | 717 | |
| 27 | ILLGPADSF | 5.6526 | 1010 | 5.50208 |
| 28 | LNPSVAATL | 5.6281 | 1254 | |
| 29 | GLLSFLVFF | 5.6226 | 764 | |
| 30 | YVYDHLTPL | 5.6148 | 948 | |
| 31 | HYAPRPCGI | 5.5954 | 488 | |
| 32 | GUHLHRNI | 5.595 | 688 | |
| 33 | HYRDVLKEM | 5.5928 | 2482 | |
| 34 | YYKVFLARL | 5.5825 | 834 | 7.24746 |
| 35 | CMVDYPYRL | 5. 566 | 607 | |
| 36 | AVIPDREVL | 5.5541 | 1693 | |
| 37 | NFSRCWVAL | 5.5313 | 234 | 6.28275 |
| 38 | VFSDMETKL | 5.5307 | 975 | 7,30704 |
| 39 | VWPLLLLLL | 5.5297 | 793 | |
| 40 | ITYSTYCKF | 5.5171 | 1291 | 6,97507 |
| 41 | IEPLDLPQI | 5.5049 | 2873 | |
| 42 | LLSTTEWQI | 5.4989 | 666 | 8.33563 |
| 43 | PLLREEVVF | 5.4208 | 2139 | |
| 44 | ATPPGSITV | 4.2466 | 1349 | |

The sequences of SEQ ID NOS: 1 to 44 are sequences consisting of 9 amino acid residues contained in a certain genome protein (SEQ ID NO: 184) of the HCV D90208 strain, which will be described later. The sequences of SEQ ID NOS: 1 to 44 are sequences predicted by the above-mentioned method to have superior binding to an HLA-A24 type molecule. SEQ ID NOS: 1 to 44 are arranged in decreasing binding order. That is, SEQ ID NO: 1 is the sequence that is predicted to have the best binding. A predicted score for binding to the HLA-A24 type molecule and binding experiment data for each sequence are expressed in the form of -log Kd values.

The sequences of SEQ ID NOS: 45 to 83 are given in Table 2 below.

**TABLE. 2 HLA - AA24 - BINDING PEPTIDE**

| SEQ ID NO | D89815 PREDICTED SCORE | PREDICTED SCORE | SEQ NAME | BINDING EXPERIMENT DATA |
|---|---|---|---|---|
| 45 | VILDSPEPL | 6.4276 | 2251 | 5.00343 |
| 46 | ILPCSYTTL | 6.131 | 674 | |
| 47 | FWAKHMWNF | 6.0822 | 1760 | |
| 48 | ALYDVVSTL | 6.0484 | 2593 | 6.38942 |
| 49 | AFYGVWPLL | 5.9676 | 789 | 7.7344 |
| 50 | PYIEQGMQL | 5.9628 | 1716 | |
| 51 | TPPAVPQTF | 5.9302 | 1215 | |
| 52 | WHYPCTVNF | 5.921 | 616 | 6.38729 |
| 53 | GILPFFMFF | 5.9182 | 764 | 7.69551 |
| 54 | GLIHLHQNI | 5. 879 | 688 | 5.85566 |
| 55 | LMCAVHPEL | 5.8442 | 876 | 6.59126 |
| 56 | TVLADFKTW | 5.8411 | 1986 | 6.51874 |
| 57 | AYSQQTRGL | 5.831 | 1031 | |
| 58 | AQPGYPWPL | 5.8258 | 77 | 5.36419 |
| 59 | PLLRDEVTF | 5.8128 | 2139 | 5.08926 |
| 60 | ILMTHFFSI | 5.8071 | 2843 | 7,89519 |
| 61 | SYTWTGALI | 5.8059 | 2422 | 7.12954 |
| 62 | ATPPGSVTF | 5.7779 | 1349 | 6.51124 |
| 63 | LLPRRGPRL | 5. 7503 | 36 | 7.71195 |
| 64 | LMTHFFSIL | 5.7443 | 2844 | 5.9169 |
| 65 | LLKRLHQWI | 5.7425 | 1956 | 6.85724 |
| 66 | ARPDYNPPL | 5.7226 | 2289 | |
| 67 | AYYSMVGNW | 5.7076 | 360 | 6.46991 |
| 68 | KFPAAMPVW | 5.7062 | 2280 | |
| 69 | QYTLLFNIL | 5.7028 | 1804 | |
| 70 | LVPGAAYAF | 5.6865 | 782 | |
| 71 | RYAPACKPL | 5.6851 | 2132 | 6.75756 |
| 72 | FLARLIWWL | 5.6847 | 838 | 6.17696 |
| 73 | SQLDLSGWF | 5.6728 | 2962 | |
| 74 | SMLTDPSHI | 5.6657 | 2173 | 6.94014 |
| 75 | ILLGPADSF | 5.6526 | 1010 | 5.50208 |
| 76 | WLRDVWDWI | 5.6315 | 1976 | 6.34379 |
| 77 | YVVLLFLLL | 5.6308 | 718 | |
| 78 | LNPSVAATL | 5.6281 | 1254 | |
| 79 | YVYDHLTPL | 5.6148 | 948 | |
| 80 | HYRDVLKEM | 5.5928 | 2482 | |
| 81 | TLRRHVDLL | 5.5762 | 257 | |
| 82 | AVIPDREVL | 5.5541 | 1693 | |
| 83 | FLISQLFTF | 5.5528 | 285 | |

The sequences of SEQ ID NOS: 45 to 83 are sequences consisting of 9 amino acid residues contained in a certain genome protein (SEQ ID NO: 185) of the HCV D89815 strain. The sequences of SEQ ID NOS: 45 to 83 are sequences predicted by the above-mentioned method to have superior binding to an HLA-A24 type molecule. SEQ ID NOS: 45 to 83 are arranged in decreasing binding order. That is, SEQ ID NO: 45 is the sequence that is predicted to have the best binding. A predicted score for binding to the HLA-A24 type molecule and binding experiment data for each sequence are expressed in the form of -log Kd values.

The sequences of SEQ ID NOS: 84 to 123 are shown in Table 3 below.

**TABLE. 3 HLA - A24- BINDING PEPTIDE**

| SEQ ID NO | pBRT703' X PREDICTED SCORE | PREDICTED SCORE | SEQ NAME | BINDING EXPERIMENT DATA |
|---|---|---|---|---|
| 84 | VILDSFEPL | 6.7012 | 2251 | 5.00343 |
| 85 | ILPCSYTTL | 6.2441 | 674 | |
| 86 | GILPFFMFF | 6.1234 | 764 | 7.69551 |
| 87 | PLLRDEVTF | 6.0954 | 2139 | 5.08826 |
| 88 | TPPAVPQTF | 6.0934 | 1215 | |
| 89 | FWAKHMWNF | 6.0822 | 1760 | |
| 90 | TVLADFKTW | 5.9355 | 1986 | 6.51874 |
| 91 | ALYDVVSTL | 5.9179 | 2593 | 6.38942 |
| 92 | WHYPCTVNF | 5.8742 | 616 | 6.38729 |
| 93 | ATPPGSVTF | 5.8681 | 1349 | 6.51124 |
| 94 | GLIHLHQNI | 5.8476 | 688 | 5.85566 |
| 95 | AYSQQTRGL | 5.831 | 1031 | |
| 96 | ILMTHFFSI | 5.8217 | 2843 | 7.89519 |
| 97 | FLARLIWWL | 5.7815 | 838 | 6.17696 |
| 98 | AFYGVWPLL | 5.7373 | 789 | 7.7344 |
| 99 | FLISQLFTF | 5.7341 | 285 | |
| 100 | ILLGPADSF | 5.719 | 1010 | 5.50208 |
| 101 | SMLTDPSHI | 5.6922 | 2173 | 6.94014 |
| 102 | AYYSMVGNW | 5.6746 | 360 | 6.46991 |
| 103 | QYTLLFNIL | 5.6682 | 1804 | |
| 104 | LLKRLHQWI | 5.6343 | 1956 | 6.85724 |
| 105 | SQLDLSGWF | 5.5993 | 2962 | |
| 106 | WLRDVWDWI | 5.5818 | 1976 | 6.34379 |
| 107 | ITYSTYGKF | 5.5352 | 1291 | 6.37373 |
| 108 | SYTWTGALI | 5.5253 | 2422 | 7.12954 |
| 109 | LLSTTEWQI | 5.5182 | 666 | 8.33563 |
| 110 | RYAPACKPL | 5.5076 | 2132 | 6.75756 |
| 111 | RLIWWLQYF | 5.5035 | 841 | |
| 112 | VLADFKTWL | 5.4871 | 1987 | 6.63423 |
| 113 | LVPGAAYAF | 5.4661 | 782 | |
| 114 | DLPQIIQRL | 5.4605 | 2877 | |
| 115 | WICTVLADF | 5.4521 | 1983 | |
| 116 | FYGVWPLLL | 5.4409 | 790 | |
| 117 | LLLSILGPL | 5.4365 | 891 | |
| 118 | HYRDVLKEM | 5.4331 | 2482 | |
| 119 | LIWWLQYFI | 5.4328 | 842 | |
| 120 | AVIPDREVL | 5.4247 | 1693 | |
| 121 | TRPPHGNWF | 5.4243 | 542 | |
| 122 | KFPAAMPVW | 5.424 | 2280 | |
| 123 | VFPDLGVRV | 5.3898 | 2580 | 6.73918 |

The sequences of SEQ ID NOS: 84 to 123 are sequences consisting of 9 amino acid residues contained in a certain genome protein (SEQ ID NO: 186) of the HCV pBRT703'X strain, which will be described later. The sequences of SEQ ID NOS: 84 to 123 are sequences predicted by the above-mentioned method to have superior binding to an HLA-A24 type molecule. SEQ ID NOS: 84 to 123 are arranged in decreasing binding order. That is, SEQ ID NO: 84 is the sequence that is predicted to have the best binding. A predicted score for the binding to the HLA-A24 type molecule and binding experiment data for each sequence are expressed in the form of -log Kd values.

The sequences of SEQ ID NOS: 124 to 183 are shown in Table 4 below.

**TABLE. 4 HLA - A2 - BINDING PEPTIDE**

| SEQ ID NO | pBRT703' X PREDICTED SCORE | PREDICTED SCORE | SEQ NAME | BINDING EXPERIMENT DATA |
|---|---|---|---|---|
| 124 | KLLPRLPGV | 5.9316 | 1998 | 6.70726 |
| 125 | DMPSTEDLV | 5.925 | 1872 | |
| 126 | YLYGIGSAV | 5.8812 | 701 | 5.56617 |
| 127 | YLNTPGLPV | 5.7437 | 1542 | 5.67247 |
| 128 | CLLLLSVGV | 5.7302 | 2994 | |
| 129 | LLLSVGVGI | 5.6528 | 2996 | |
| 130 | LLCPSGHVV | 5.6238 | 1169 | |
| 131 | AILSPGALV | 5.6128 | 1885 | 6.24349 |
| 132 | SLIRVPYFV | 5.5906 | 805 | 5.86269 |
| 133 | DVWDWICTV | 5.5657 | 1979 | 4.87956 |
| 134 | VIPASGDVV | 5.558 | 1426 | 6.24145 |
| 135 | RALAHGVRV | 5.5481 | 149 | 5.28381 |
| 136 | LSDGSWSTV | 5.5257 | 2400 | 6.22313 |
| 137 | KLQDCTMLV | 5.4922 | 2726 | 5.25202 |
| 138 | YCLTTGSVV | 5.4899 | 1873 | |
| 138 | SMLTDPSHI | 5.4885 | 2173 | 5.55941 |
| 140 | AAFCSAMYV | 6.4454 | 269 | |
| 141 | YSPGEINRV | 5.4058 | 2896 | 6.65123 |
| 142 | YTNVDODLV | 5.4046 | 1101 | 5.67802 |
| 143 | LRDEVTFQV | 5.4015 | 2141 | |
| 144 | LAALTGTYV | 5.3812 | 941 | |
| 145 | CEPEPDVTV | 5.3645 | 2162 | |
| 146 | CMSADLEVV | 5.3561 | 1648 | 4.80983 |
| 147 | VFPDLGVRV | 5.3546 | 2580 | 6.02403 |
| 148 | YCFTPSPVV | 5.3206 | 507 | |
| 149 | VLQASLIRV | 5.2832 | 901 | 5.46327 |
| 150 | KQAEAAAPV | 5.2819 | 1741 | 5.41584 |
| 151 | LLLALPPRA | 5.2558 | 799 | |
| 152 | VLDDHYRDV | 5.2542 | 2478 | 6.51164 |
| 153 | FSPRRHETV | 5.2377 | 293 | |
| 154 | SVIDCNTCV | 5.2251 | 1450 | |
| 155 | GLIRACTLV | 5.1743 | 917 | |
| 156 | TVNFTIFKV | 5.1707 | 621 | |
| 157 | EMGGNITRV | 5.1651 | 2236 | |
| 158 | PLLRHHNMV | 5.1643 | 2448 | |
| 159 | QLDLSGWFV | 5.1635 | 2963 | |
| 160 | TLAARNASV | 5.1583 | 245 | |
| 161 | RLGAVQNEV | 5.1386 | 1627 | |
| 162 | VILDSFEPL | 5.138 | 2251 | 5.38729 |
| 163 | AALENLVVL | 5.1347 | 746 | |
| 164 | LLEDTDTPI | 5.1223 | 2545 | |
| 165 | VVTSTWVLV | 5.1189 | 1655 | |
| 166 | FSLDPTFTI | 5.1183 | 1464 | |
| 167 | TIPASAYEV | 5.1158 | 188 | |
| 168 | DLLEDTDTP | 5.081 | 2544 | |
| 169 | LLLSILGPL | 5.0753 | 891 | |
| 170 | VLADFKTWL | 5.0725 | 1987 | 6.01696 |
| 171 | SILGIGTVL | 5.071 | 1325 | |
| 172 | AGDNFPYLV | 5.0651 | 1579 | |
| 173 | ILPCSYTTL | 5.0643 | 674 | 6.37008 |
| 174 | VAAEEYVEV | 5.0509 | 2085 | |
| 175 | LAVAVEPVV | 5.0484 | 967 | |
| 176 | ALYDVVSTL | 5.0301 | 2593 | 6.14967 |
| 177 | FLARLIWWL | 5.0259 | 838 | 5.67557 |
| 178 | RLLAPITAY | 5.0244 | 1024 | |
| 179 | WLRDVWDWI | 5.0191 | 1976 | 5.68156 |
| 180 | CVWGACWTV | 5.0181 | 1073 | |
| 181 | YVYDHLTPL | 5.0087 | 948 | |
| 182 | TVVLTESTV | 5.0061 | 2332 | |
| 183 | AARALAHGV | 5.0044 | 147 | |

The sequences of SEQ ID NOS: 124 to 183 are sequences consisting of 9 amino acid residues contained in a certain genome protein (SEQ ID NO: 186) of the HCV pBRT703'X strain, which will be described later. The sequences of SEQ ID NOS: 124 to 183 are sequences predicted by the above-mentioned method to have superior binding to an HLA-A2 type molecule. SEQ ID NOS: 124 to 183 are arranged in decreasing binding order. That is, SEQ ID NO: 124 is the sequence that is predicted to have the best binding. A predicted score for the binding to the HLA-A2 type molecule and binding experiment data for each sequence are expressed in the form of -log Kd values.

Although details are described later, it is clear that in all of Table 1 to Table 4, there is a correlation between the predicted score and the binding experiment data. That is, although there are slight errors, it can be said that a peptide that is predicted by the above-mentioned method to have high binding to the HLA-A type molecule is found experimentally to have high binding to the HLA-A type molecule.

Since there is no conventional technique for discovering an HLA-binding peptide by utilizing such an experimental design method, there are only a very small number of HLA-binding peptides that have been experimentally confirmed to have HLA-binding properties. Because of this, even when a peptide consisting of 9 amino acid residues is randomly synthesized by a conventional method, and subjected to an experiment to find out if it binds to an HLA molecule, there is a probability of only about 1 in 100 of finding one that has a binding in terms of a -log Kd value, exceeding 6.

In accordance with this embodiment, since the technique of finding an HLA-binding peptide by utilizing the experimental design method is used, as described above, as many as 183 sequences of HLA-binding peptides can be found. Furthermore, when the binding of some of the HLA-binding peptides obtained is experimentally examined, it is confirmed that all of the sequences that have been subjected to the experiment exhibit an excellent binding to HLA that is equal to or higher than that predicted.

Among these sequences, an HLA-binding peptide containing at least one type of amino acid sequence selected from the group consisting of SEQ ID NOS: 1, 2, 3, 5, 8, 12, 13, 14, 16, 17, 18, 19, 22, 23, 25, 27, 34, 37, 38, 40, 42, 45, 48, 49, 52, 53, 54, 55, 56, 58, 59, 60, 61, 62, 63, 64, 65, 67, 71, 72, 74, 75, 76, 84, 86, 87, 90, 91, 92, 93, 94, 96, 97, 98, 100, 101, 102, 104, 106, 107, 108, 109, 110, 112, 123, 124, 126, 127, 131, 132, 133, 134, 135, 136, 137, 139, 141, 142, 146, 147, 149, 150, 152, 162, 170, 173, 176, 177, and 179 is experimentally confirmed to bind to a human HLA-A type molecule. It can therefore be said with certainty that it is an HLA-binding peptide that has excellent properties in binding to a human HLA-A type molecule.

The binding to an HLA molecule of the HLA-binding peptide related to the present embodiment is 3 or greater in term of a -log Kd value, particularly preferably 5 or greater, and more preferably 5.4 or greater.

In the field of biochemistry, it is known that a binding ability in terms of a -log Kd value, of about 3 is the threshold level for whether or not a peptide actually binds to an MHC such as an HLA. Therefore, if the binding to an HLA molecule in terms of a -log Kd value, is 3 or greater, it can be said that it is an HLA-binding peptide.

Furthermore, if the binding to an HLA molecule in terms of a -log Kd value, is 5 or greater, since the peptide obtained has excellent properties in binding to the HLA molecule, it can suitably be used for development of an effective therapeutic drug, prophylactic drug and the like for an immune disease and the like.

Moreover, if the binding to an HLA molecule in terms of a -log Kd value, is 5.4 or greater, the peptide obtained has particularly good properties in binding to the HLA molecule, and it can suitably be used for the development of an even more effective therapeutic drug, preventive drug and the like for an immune disease and the like.

Furthermore, it may be arranged that the HLA-binding peptide related to the present embodiment consists of not less than 8 and not more than 11 amino acid residues.

In this way, if the peptide consists of not less than 8 and not more than 11 amino acid residues, it has excellent properties in binding to an HLA molecule. Furthermore, the cytotoxic T lymphocyte (CTL) specifically recognizes a virus antigen (CTL epitope) consisting of 8 to 11 amino acids presented in an HLA class I molecule on the surface of a cell infected with a virus and the like, and eliminates the virus by damaging the infected cell. It is important to prepare such a CTL epitope consisting of 8 to 11 amino acids that is specific to a virus and the like in order to prepare a vaccine for therapy or prevention against the virus and the like.

For example, the above-mentioned HLA-binding peptide may be a peptide consisting of amino acid residues alone, but it is not particularly limited thereto. For example, it may be an HLA-binding peptide precursor that is optionally modified with a sugar chain or a fatty acid group or the like as long as the effects of the present invention are not impaired. Such a precursor is subjected to a change involving digestion by a digestive enzyme and the like in a living mammalian body such as in a human digestive organ to become an HLA-binding peptide, thus exhibiting the similar effects to those shown by the above-mentioned HLA-binding peptide.

Furthermore, the above-mentioned HLA-binding peptide may be a peptide that binds to a human HLA-A24 type molecule.

Moreover, the above-mentioned HLA-binding peptide may be a peptide that binds to a human HLA-A2 type molecule.

In accordance with this constitution, since a peptide is obtained that binds to an HLA-A24 type molecule, which is often seen in Asian people, such as Japanese people, it can be utilized in the development of a therapeutic drug, a preventive drug and the like that is particularly effective for Asian people, such as Japanese people.

Furthermore, in accordance with this constitution, since a peptide is obtained that binds to an HLA-A2 type molecule, which is often seen in European and American people in addition to Japanese people, it can be utilized in the development of a therapeutic drug, a preventive drug and the like that is particularly effective for European and American people in addition to Japanese people.

The amino acid sequence contained in the above-mentioned HLA-binding peptide may be an amino acid sequence derived from a certain genome protein of HCV, but it is not particularly limited thereto. For example, it may be an amino acid sequence derived from an HIV protein, an amino acid sequence derived from a cedar pollen protein and the like. Moreover, it may contain an amino acid sequence derived from a protein having the other pathogenicity or allergenicity.

For example, when it contains an amino acid sequence derived from an HCV envelope protein, an HLA-binding peptide that can be utilized in the prevention, therapy and the like of a disease caused by HCV can be obtained.

### <Embodiment 2>

In accordance with this embodiment, there is provided an HLA-binding peptide that binds to an HLA-A type molecule, contains an amino acid sequence formed by deletion, substitution, or addition of one or two amino acid residues of the amino acid sequence contained in the above-mentioned HLA-binding peptide, and consists of not less than 8 and not more than 11 amino acid residues.

As described later, even though the constitution includes an amino acid sequence formed by deletion, substitution, or addition of one or a few amino acid residues of a specific amino acid sequence that binds to an HLA-A type molecule, the similar effects to those of the HLA-binding peptide related to the above-mentioned embodiment 1 are exhibited.

Although the amino acid sequences of polyproteins of the above-mentioned HCV D90208 strain, D89815 strain, and pBRT703'X strain (mutant subclone of D89815) are different from each other in part, since the correlation between predicted data and experimental data for the -log Kd value of several 9-mer peptides existing in a certain genome protein of the D90208 strain is high, that is, a sequence that is determined to be binding based on predicted data shows a good -log Kd value in experimental data, it can be predicted that the D89815 strain and the pBRT703'X strain (mutant subclone of D89815) will show a -log Kd value with a superior ranking in the predicted data. Therefore, it can be predicted that even amino acid sequences in a certain genome proteins of the D89815 strain and the pBRT703'X strain (mutant subclone of D89815), which are amino acid sequences formed by substitution of one or two amino acid residues of the amino acid sequences that exhibit binding properties, will similarly show excellent HLA-binding properties.

That is, it can be predicted that even an amino acid sequence formed by deletion, substitution, or addition of one or two amino acid residues of an amino acid sequence shown in SEQ ID NOS: 1 to 183 that has excellent properties in binding to an HLA-A type molecule will show excellent HLA-binding properties in the similar manner.

From another viewpoint, it can be predicted that even an amino acid sequence formed by deletion, substitution, or addition of one or a few amino acid residues of an amino acid sequence predicted by the above-mentioned method to have excellent properties in binding to an HLA-A type molecule will show excellent HLA-binding properties in the similar manner. The amino acid residues that are substituted are preferably amino acid residues having similar properties to each other, such that both are hydrophobic amino acid residues.

Moreover, the HLA-binding peptides described in Embodiment 1 and Embodiment 2 can be produced using a method known to a person skilled in the art. For example, they may be artificially synthesized by a solid-phase method or a liquid-phase method. Alternatively, these HLA-binding peptides may be produced by expressing them from a DNA segment or a recombinant vector coding for these HLA-binding peptides. These HLA-binding peptides thus obtained can be identified by a method known to a person skilled in the art. For example, identification is possible by use of Edman degradation, mass spectrometry and the like.

### <Embodiment 3>

In accordance with the present embodiment, there is provided a DNA segment containing a DNA sequence coding for the above-mentioned HLA-binding peptide. Since the DNA segment related to the present embodiment contains a specific DNA sequence, it can express the above-mentioned HLA-binding peptide.

When the above-mentioned HLA-binding peptide is expressed by using the DNA segment related to the present embodiment, expression may be carried out by incorporating this DNA segment into a cell, or expression may be carried out by using a commercial artificial protein expression kit.

Furthermore, continuous expression may be carried out by incorporating the above-mentioned DNA segment into, for example, a human cell. Because of this, an HLA-binding peptide can be made to be present constitutively within a cell by incorporating a DNA segment coding for the HLA-binding peptide into the cell rather than incorporating the HLA-binding peptide itself into the cell. When an HLA-binding peptide is used as a vaccine, such an ability to express constitutively is advantageous in terms of enhancing the efficacy of the vaccine.

Moreover, the DNA segment related to the present embodiment can be produced by a method known to a person skilled in the art. For example, it may be artificially synthesized by means of a commercial DNA synthesizer and the like. Alternatively, it may be segmented from the HCV genome by using a restriction enzyme and the like. Alternatively, it may be amplified from the HCV genome by a PCR method using a pair of primers. The DNA segment thus obtained may be identified using a method known to a person skilled in the art. For example, it may be identified by a commercial DNA sequencer.

### <Embodiment 4>

In accordance with the present embodiment, there is provided a recombinant vector that contains a DNA sequence coding for the above-mentioned HLA-binding peptide. Since the recombinant vector related to the present embodiment contains a specific DNA sequence, the above-mentioned HLA-binding peptide can be expressed.

When the above-mentioned HLA-binding peptide is expressed by using the recombinant vector related to the present embodiment, expression may be carried out by incorporating this recombinant vector into a cell, or expression may be carried out by using a commercial artificial protein expression kit.

Furthermore, continuous expression may be carried out by incorporating the above-mentioned recombinant vector into, for example, a human cell. Because of this, the HLA-binding peptide can be made to be present continuously within a cell by incorporating a recombinant vector coding for the HLA-binding peptide into the cell rather than incorporating the HLA-binding peptide itself into the cell. When the HLA-binding peptide is used as a vaccine, such an ability to express continuously is advantageous in terms of enhancing the efficacy of the vaccine.

Furthermore, in the above-mentioned recombinant vector, the amount of HLA-binding peptide expressed can be controlled with high precision by the use of a certain sequence in a regulatory region involved in transcription and expression, such as a promoter region upstream of a DNA sequence coding for the above-mentioned HLA-binding peptide. Moreover, the number of copies of a recombinant vector in a cell can be controlled with high precision by the use of a certain sequence in a regulatory region involved in replication, such as the origin region of the recombinant vector.

Furthermore, the above-mentioned recombinant vector may freely contain a sequence other than the DNA sequence coding for the above-mentioned HLA-binding peptide. For example, it may contain a sequence of a marker gene such as a drug resistance gene.

Moreover, the recombinant vector related to the present embodiment can be produced using a method known to a person skilled in the art. For example, it may be obtained by cleaving a multicloning site of a commercial vector such as pBR322 or pUC19 at a certain restriction enzyme site, and inserting the above-mentioned DNA segment into the site and carrying out ligation. Furthermore, the recombinant vector thus obtained can be identified using a method known to a person skilled in the art. For example, it can be confirmed by agarose gel electrophoresis whether or not the length of the DNA segment cleaved by a predetermined restriction enzyme coincides with the restriction map of a commercial vector such as pBR322 or pUC19 and, furthermore, it can be identified by a DNA sequencer and the like whether or not the above-mentioned DNA sequence is contained in the DNA sequence cut out from the multicloning site.

Although embodiments of the present invention are described above, they are illustrated as examples of the present invention, and various constitutions other than the above may be employed.

For example, in the above-mentioned embodiments, the HLA-binding peptide contains an amino acid sequence derived from a certain genome protein (SEQ ID NOS: 184, 185, 186) of HCV, but an HLA-binding peptide containing an amino acid sequence derived from another HCV protein may be used. In such a case, it can be used for the therapy of various types of immune diseases related to the protein from which it is derived.

Furthermore, an HLA-binding peptide for a pathogen other than HCV, such as an HIV virus, may be employed, and an HLA-binding peptide containing an amino acid sequence derived from a protein such as a cedar pollen allergen and the like, or a cancer cell may be employed.

In this way, if an amino sequence that is predicted by the above-mentioned method to have excellent HLA-binding properties is contained, it can be expected that it will exhibit excellent HLA-binding properties in the similar way when confirmation is carried out experimentally. Because of this, these HLA-binding peptides can suitably be used mainly for the therapy or prevention of infectious diseases (influenza, SARS, HIV, HCV and the like), and also for cancer immunotherapy, allergic diseases (pollen allergy (hay fever), rheumatism, atopy, asthma and the like), autoimmune diseases and the like.

### (Examples)

The present invention is further explained below by reference to Examples, but the present invention is not limited thereto.

Specifically, procedures of prediction, experiment, and evaluation in the present examples were carried out based on an active learning experiment design, and in general the following steps were repeated. A schematic drawing for the active learning experiment design employed here is shown in FIG. 1.
(1) A trial of a lower-order learning algorithm, which will be described later, was carried out once. That is, a plurality of hypotheses were generate by random sampling from accumulated data and, with regard to randomly expressed candidate query points (peptide), a point that showed the largest distribution of predicted values was selected as a query point to be subjected to an experiment.
(2) The peptide at the selected query point was prepared by a synthesis and purification method, which will be described later, and the actual binding ability was measured by an experiment, which will be described later, and added to accumulated data.

In the present example, as the lower-order learning algorithm, a supervised learning algorithm of a Hidden Markov Model was used, and 20 to 30 types of peptides were predicted and selected per experiment by starting with the initial data for 223 types of peptides; the above-mentioned procedure was repeated four times, and a total of 341 data points were obtained.

More specifically, in the active learning method of the present example, 20 to 30 types of peptides containing an amino acid sequence in which 9 of 20 types of amino acids were arranged were designed and synthesized per experiment. The strength of binding (binding ability) thereof to an HLA molecule was measured. The binding ability (Kd value in molar concentration) was obtained as an experimental result. When the binding ability was high, the peptide was selected as a candidate for an HLA-binding peptide that could be used as a material for a vaccine.

The results thus obtained were inputted into a learning system equipped with a learning machine employing the Hidden Markov Model as a mathematical algorithm, and rules were created. The learning machine sampled different results to prepare the rules. The rules expressed by the learning machine had different constitutions. The rules thus obtained and experimental data were stored as needed as accumulated data.

From among more than 20⁹ = 500 billion peptide sequences, candidates for a subsequent experiment were selected by the rules, and the above-mentioned process was repeated. In this stage, different rules were applied to experimental candidates, and the candidates for which predictions of the experimental results were divided were subjected to experiment. In this way, since the candidates for which predictions of the experimental results were divided were subjected to subsequent experiment, the final precision of the prediction was increased.

In this way, a plurality of learning machines carried out selective sampling in which samples that would give different predictions were selected as experimental candidates, information could be gained efficiently, and a hypothesis (rule) with high precision could be obtained. Repeating the above-mentioned process four times gave excellent results as in Examples described later. Repeating it seven times or more gave even better results.

In accordance with such an active learning method, the number of repetitions of the binding experiment for peptides consisting of 9 amino acid residues, which would otherwise have to be carried out for the 500 billion or more combinations of all the candidates for HLA-binding peptides, could be reduced. In the active learning method, a rule was formed by experiment, and the experiment was repeated for tens of sequence candidates that were predicted by applying the rule. Because of this, the number of experiments could be cut, and the time and cost of the initial screening could be greatly reduced.

Furthermore, the hit rate for prediction of the binding of a peptide to HLA by the rule obtained by the active learning method reached 70 to 80%, whereas the hit rate by other known techniques such as the anchor method was as low as about 30%.

### <Synthesis and purification of peptide>

A peptide was manually synthesized by the Merrifield solid-phase method using Fmoc amino acids. After deprotection, reverse phase HPLC purification was carried out using a C18 column to give a purity of 95% or higher. Identification of the peptide and confirmation of its purity were carried out using a MALDI-TOF mass spectrometer (Voyager DE RP, PerSeptive). Quantitative analysis of the peptide was carried out by a Micro BCA assay (Pierce Corp.) using BSA as a standard protein.

### <Experiment of binding peptide to HLA-A24 type molecule>

The ability of a peptide to bind to an HLA-A24 type molecule, which is a product of the HLA-A*2402 gene, was measured using C1R-A24 cells expressing the HLA-A24 type molecule (cells prepared by Professor Masafumi Takiguchi, Kumamoto University being supplied with permission by Assistant Professor Masaki Yasukawa, Ehime University).

C1R-A24 cells were first exposed to acidic conditions at a pH of 3.3 for 30 seconds, thus dissociating and removing a light chain β2m, which is associated with HLA class I molecules in common, and an endogenous peptide originally bound to the HLA-A*2402 molecule. After neutralization, purified β2m was added to C1R-A24 cells, and the obtained product was added to serial dilutions of a peptide, and incubated on ice for 4 hours. Staining was carried out using fluorescently labeled monoclonal antibody 17A12, which recognizes association (MHC-pep) of the three members, that is, HLA-A*2402 molecule, the peptide, and β2m, which had reassociated during the incubation.

Subsequently, the MHC-pep count per C1R-A24 cell (proportional to the strength of fluorescence of the above-mentioned fluorescent antibody) was quantitatively measured using an FACScan fluorescence-activated cell sorter (Becton Dickinson Biosciences). A binding dissociation constant Kd value between the HLA-A24 type molecule and the peptide was calculated from the average strength of fluorescence per cell by a published method (Udaka et al., Immunogenetics, 51, 816-828, 2000).

### <Experiment of binding peptide to HLA-A2 type molecule>

The ability of a peptide to bind to an HLA-A2 type molecule, which is a product of the HLA-A*0201 gene, was measured using strain JY cells expressing the HLA-A*0201.

JY cells were first exposed to acidic conditions at a pH of 3.8 for 30 seconds, thus dissociating and removing a light chain β2m and an endogenous peptide, which were noncovalently associated with the HLA-A*0201 molecule. After neutralization, a reassociation experiment was carried out.

The above-mentioned JY cells and the purified β2m were added to stepped serial dilutions of peptide for which the binding ability would be measured, and incubation was carried out on ice for 4 hours. HLA-A*0201 molecules that had reassociated up to this point were stained using the associating type specific fluorescently-labeled monoclonal antibody BB7.2.

Subsequently, the amount of fluorescence per a cell was measured using a flow cytometer and a dissociation constant Kd value in molar concentration was calculated by a published method (Udaka et al., Immunogenetics, 51, 816-828, 2000).

### <Evaluation results>

The prediction results and the experimental results shown in Tables 1 to 4 above were obtained.

The sequences of SEQ ID NOS: 1 to 44 in Table 1 are sequences consisting of 9 amino acid residues contained in the full-length sequence of a certain genome protein of the HCV D90208 strain registered in the GenBank. Furthermore, the sequences of SEQ ID NOS: 1 to 44 are sequences having superior binding to an HLA-A24 type molecule as predicted by a hypothesis obtained by the experimental design method explained in Embodiment 1. SEQ ID NOS: 1 to 44 are arranged in decreasing binding order. That is, SEQ ID NO: 1 is the sequence that is predicted to have the best binding. The full-length amino acid sequence of the certain genome protein of the HCV D90208 strain is shown in SEQ ID NO: 184

(MSTNPKPQRKTKRNTNRRPQDVKFPGGGQIVGGVYLLPRRGPRLGVRATRKTSERS
QPRGRRQPIPKARRPEGRTWAQPGYPWPLYGNEGMGWAGWLLSPRGSRPSWGPTDPR
RRSRNLGKVIDTLTCGFADLMGYIPLVGAPLGGAARALAHGVRVLEDGVNYATGNLP
GCSFSIFLLALLSCLTIPASAYEVRNVSGIYHVTNDCSNSSIVYEAADMIMHTPGCV
PCVRESNFSRCWVALTPTLAARNSSIPTTTIRRHVDLLVGAAALCSAMYVGDLCGSV
FLVSQLFTFSPRRYETVQDCNCSIYPGHVSGHRMAWDMMMNWSPTTALVVSQLLRIP
QAVVDMVAGAHWGVLAGLAYYSMVGNWAKVLIVMLLFAGVDGHTHVTGGRVASSTQS
LVSWLSQGPSQKIQLVNTNGSWHINRTALNCNDSLQTGFIAALFYAHRFNASGCPER
MASCRPIDEFAQGWGPITHDMPESSDQRPYCWHYAPRPCGIVPASQVCGPVYCFTPS
PVVVGTTDRFGAPTYSWGENETDVLLLSNTRPPQGNWFGCTWMNSTGFTKTCGGPPC
NIGGVGNNTLVCPTDCFRKHPEATYTKCGSGPWLTPRCMVDYPYRLWHYPCTVNFTV
FKVRMYVGGVEHRLNAACNWTRGERCDLEDRDRSELSPLLLSTTEWQILPCSFTTLP
ALSTGLIHLHRNIVDVQYLYGIGSAVVSFAIKWEYILLLFLLLADARVCACLWMMLL
IAQAEATLENLVVLNAASVAGAHGLLSFLVFFCAAWYIKGRLVPGAAYALYGVWPLL

LLLLALPPRAYAMDREMAASCGGAVFVGLVLLTLSPYYKVFLARLIWWLQYFITRAE
AHLQVWVPPLNVRGGRDAIILLTCAVHPELIFDITKLLLAILGPLMVLQAGITRVPY
FVRAQGLIRACMLVRKVAGGHYVQMAFMKLAALTGTYVYDHLTPLRDWAHAGLRDLA
VAVEPVVFSDMETKLITWGADTAACGDIISGLPVSARRGKEILLGPADSFGEQGWRL
LAPITAYSQQTRGLLGCIITSLTGRDKNQVDGEVQVLSTATQSFLATCVNGVCWTVY
HGAGSKTLAGPKGPITQMYTNVDQDLVGWPAPPGARSMTPCTCGSSDLYLVTRHADV
VPVRRRGDSRGSLLSPRPISYLKGSSGGPLLCPSGHVVGIFRAAVCTRGVAKAVDFI
PVESMETTMRSPVFTDNSSPPAVPQTFQVAHLHAPTGSGKSTKVPAAYAAQGYKVLV
LNPSVAATLGFGAYMSKAHGIEPNIRTGVRTITTGGPITYSTYCKFLADGGCSGGAY
DIIICDECHSTDSTTILGIGTVLDQAETAGARLVVLATATPPGSITVPHPNIEEVAL
SNTGEIPFYGKAIPIEAIKGGRHLIFCHSKKKCDELAAKLTGLGLNAVAYYRGLDVS
VIPTSGDVVVVATDALMTGFTGDFDSVIDCNTCVTQTVDFSLDPTFTIETTTLPQDA
VSRAQRRGRTGRGRSGIYRFVTPGERPSGMFDSSVLCECYDAGCAWYELTPAETSVR
LRAYLNTPGLPVCQDHLEFWESVFTGLTHIDAHFLSQTKQAGDNLPYLVAYQATVCA
RAQAPPPSWDQMWKCLIRLKPTLHGPTPLLYRLGAVQNEVTLTHPITKYIMACMSAD
LEVVTSTWVLVGGVLAALAAYCLTTGSVVIVGRIILSGRPAVIPDREVLYQEFDEME
ECASHLPYIEQGMQLAEQFKQKALGLLQTATKQAEAAAPVVESKWRALEVFWAKHMW
NFISGIQYLAGLSTLPGNPAIASLMAFTASITSPLTTQNTLLFNILGGWVAAQLAPP
SAASAFVGAGIAGAAVGSIGLGKVLVDILAGYGAGVAGALVAFKVMSGEMPSTEDLV
NLLPAILSPGALVVGVVCAAILRRHVGPGEGAVQWMNRLIAFASRGNHVSPTHYVPE
SDAAARVTQILSSLTITQLLKRLHQWINEDCSTPCSGSWLKDVWDWICTVLSDFKTW
LQSKLLPRLPGLPFLSCQRGYKGVWRGDGIMQTTCPCGAQITGHVKNGSMRIVGPKT
CSNTWHGTFPINAYTTGPCTPSPAPNYSRALWRVAAEEYVEVTRVGDFHYVTGMTTD
NVKCPCQVPAPEFFTEVDGVRLHRYAPVCKPLLREEVVFQVGLNQYLVGSQLPCEPE
PDVAVLTSMLTDPSHITAETAKRRLARGSPPSLASSSASQLSAPSLKATCTTHHDSP
DADLIEANLLWRQEMGGNITRVESENKVVILDSFDPIRAVEDEREISVPAEILRKPR
KFPPALPIWARPDYNPPLLESWKDPDYVPPVVHGCPLPSTKAPPIPPPRRKRTVVLT

ESTVSSALAELATKTFGSSGSSAVDSGTATGPPDQASDDGDKGSDVESYSSMPPLEG

EPGDPDLSDGSWSTVSGEAGEDVVCCSMSYTWTGALITPCAAEESKLPINPLSNSLL

RHHSMVYSTTSRSASLRQKKVTFDRLQVLDDHYRDVLKEMKAKASTVKARLLSIEEA

CKLTPPHSAKSKFGYGAKDVRSLSSRAVNHIRSVWEDLLEDTETPIDTTIMAKNEVF

CVQPEKGGRKPARLIVFPDLGVRVCEKMALYDVVSTLPQAVMGPSYGFQYSPGQRVE

FLVNTWKSKKCPMGFSYDTRCFDSTVTENDIRTEESIYQCCDLAPEARQAIRSLTER

LYVGGPLTNSKGQNCGYRRCRASGVLTTSCGNTLTCYLKATAACRAAKLQDCTMLVN

GDDLVVICESAGTQEDAAALRAFTEAMTRYSAPPGDPPQPEYDLELITSCSSNVSVA

HDASGKRVYYLTRDPTTPLARAAWETVRHTPVNSWLGNIIMYAPTLWARMILMTHFF

SILLAQEQLEKALDCQIYGACYSIEPLDLPQIIERLHGLSAFSLHSYSPGEINRVAS

CLRKLGVPPLRVWRHRARSVRAKLLSQGGRAATCGKYLFNWAVKTKLKLTPIPAASQ

LDLSGWFVAGYNGGDIYHSLSRARPRWFMLCLLLLSVGVGIYLLPNR).

The sequences of SEQ ID NOS: 45 to 83 are sequences consisting of 9 amino acid residues contained in the full-length sequence of a certain genome protein of the HCV D89815 strain registered in the GenBank. Furthermore, the sequences of SEQ ID NOS: 45 to 83 are sequences having superior binding to an HLA-A24 type molecule as predicted by a hypothesis obtained by the experimental design method explained in Embodiment 1. SEQ ID NOS: 45 to 83 are arranged in decreasing binding order. That is, SEQ ID NO: 45 is the sequence that is predicted to have the best binding. The full-length amino acid sequence of the certain genome protein of the HCV D89815 strain is shown in SEQ ID NO: 185

(MSTNPKPQRKTKRNTNRRPQDVKFPGGGQIVGGVYLLPRRGPRLGVRATRKTSERS
QPRGRRQPIPKARRPEGRTWAQPGYPWPLYGNEGLGWAGWLLSPRGSRPSWGPNDPR

RRSRNLGKVIDTLTCGFADLMGYIPLVGAPLGGAARALAHGVRVLEDGVNYATGNLP
GCSFSIFLLALLSCLTIPASAYEVRNVSGIYHVTNDCSNSSIVYEAADVIMHAPGCV
PCVRENNSSRCWVALTPTLAARNASVPTTTLRRHVDLLVGTAAFCSAMYVGDLCGSV
FLISQLFTFSPRRHETVQDCNCSIYPGHVSGHRMAWDMMMNWSPTAALVVSQLLRIP
QAVMDMVAGAHWGVLAGLAYYSMVGNWAKVLIVMLLFAGVDGHTRVTGGVQGHVTST
LTSLFRPGASQKIQLVNTNGSWHINRTALNCNDSLKTGFLAALFYTHKFNASGCPER
MASCRSIDKFDQGWGPITYAQPDNSDQRPYCWHYAPRQCGIVPASQVCGPVYCFTPS
PVVVGTTDRFGAPTYNWGDNETDVLLLNNTRPPHGNWFGCTWMNSTGFTKTCGGPPC
NIRGVGNNTLTCPTDCFRKHPDATYTKCGSGPWLTPRCLVDYPYRLWHYPCTVNFTI
FKVRMYVGGVEHRLDAACNWTRGERCDLEDRDRAELSPLLLSTTEWQILPCSYTTLP
ALSTGLIHLHQNIVDIQYLYGIGSAVVSIAIKWEYVVLLFLLLADARVCACLWMMLL
IAQAEAALENLVVLNAASVVGAHGMLPFFMFFCAAWYMKGRLVPGAAYAFYGVWPLL
LLLLALPPRAYAMDREMVASCGGGVFVGLALLTLSPYCKVFLARLIWWLQYFITKAE
AHLQVSLPPLNVRGGRDAIILLMCAVHPELIFDITKLLLSILGPLMVLQASLIRVPY
FVRAQGLIRACMLVRKAAGGHYVQMAFVKLAALTGTYVYDHLTPLQDWAHVGLRDLA
VAVEPVVFSAMETKVITWGADTAACGDIISGLPVSARRGKEILLGPADSFEGQGWRL
LAPITAYSQQTRGLLGCIITSLTGRDKNQVEGEVQVVSTAKQSFLATCVNGACWTVF
HGAGSKTLAAAKGPITQMYTNVDQDLVGWPAPPGARSLTPCTCGSSDLYLVTRHADV
IPVRRRGDSRGSLLSPRPISYLKGSSGGPLLCPSGHVVGIFRAAVCTRGVAKAVDFI
PVESMETTMRSPVFTDNSTPPAVPQTFQVAHLHAPTGSGKSTKVPAAYAAQGYMVLV
LNPSVAATLGFGAYMSKAHGIDPNIRTGVRTITTGAPITYSTYGKFLADGGCSGGAY
DIIICDECHSTDSTSILGIGTVLDQAETVGARFVVLATATPPGSITFPHPNIEEVPL
ANTGEIPFYAKTIPIEVIRGGRHLIFCHSKKKCDELPAKLSALGLNAVAYYRGLDVS
VIPASGDVVVVATDALMTGFTGDFDSVIDCNTCVTQTVDFSLDPTFTIETTTVPQDA
VSRTQRRGRTGRGRRGIYRFVTPGERPSAMFDSSVLCECYDAGCAWYELTPAETSVR
LRAYLNTPGLPVCQDHLEFWESVFTGLTHIDAHFLSQTKQAGDNFPYLVAYQATVCA
RAKAPPPSWDQMWKCLIRLKPTLHGPTPLLYRLGAVQNEVTLTHPITKYIMACMSAD

LEVVTSTWVLVGGVLAALAAYCLTTGSVVIVGRIILSGRPAVIPDREVLYQEFDEME
ECASHLPYIEQGMQLAEQFKQKALGLLQTATKQAEAAAPVVESKWRALETFWAKHMW
NFISGIQYLAGLSTLPGNPAIASLMAFTASITSPLATQYTLLFNILGGWVAAQLAPP
SAASAFVGAGIAGAAVGSIGLGKVLVDILAGYGAGVAGALVAFKVMSGDMPSTEDLV
NLLPAILSPGALVVGVVCAAILRRHVGPGEGAVQWMNRLIAFASRGNHVSPTHYVPE
SDAAARVTQILSNLTITQLLKRLHQWINEDCSTPCSGSWLRDVWDWICTVLADFKTW
LQSKLLPRLPGVPFFSCQRGYKGVWRGDGIMYTTCPCGAQITGHVKNGSMRIVGPRT
CSNTWHGTFPINAYTTGPCTPSPAPNYSRALWRVAAEEYVEVTRVGDFHYVTGMTTD
NVKCPCQVPAPEFFTELDGVRLHRYAPACKPLLRDEVTFQVGLNQYTVGSQLPCEPE
PDVTVVTSMLTDPSHITAEAARRRLARGSPPSLAGSSASQLSALSLKATCTTHHGAP
DTDLIEANLLWRQEMGGNITRVESENKIVILDSFEPLRAEEDEREVSAAAEILRKTR
KFPAAMPVWARPDYNPPLLESWKNPDYVPPVVHGCPLPPTKAPPIPPPRRKRTVVLT
ESTVSSALAELATKTFGGSGSSAVDSGTATGPPDQASAEGDAGSDAESYSSMPPLEG
EPGDPDLSDGSWSTVSEEASEDVVCCSMSYTWTGALITPCAAEESKLPINALSNPLL
RHHNMVYSTTSRSASLRQKKVTFDRMQVLDDHYRDVLKEMKAKASTVKAKLLSVEEA
CKLTPPHSAKSKFGYGAKDVRSLSSRAVNHIRSVWKDLLEDTDTPIQTTIMAKNEVF
CVQPEKGGRKPARLIVFPDLGVRVCEKMALYDVVSTLPQAVMGSSYGFQYSPKQRVE
FLVNTWKAKKCPMGFSYDTRCFDSTVTENDIRVEESIYQCCDLAPEARQAIRSLTER
LYIGGPMTNSKGQNCGYRRCRASGVLTTSCGNTLTCYLKAAAACRAAKLQDCTMLVC
GDDLVVICDSAGTQEDAASLRVFTEAMTRYSAPPGDPPQPEYDLELITSCSSNVSVA
HDASGKRVYYLTRDPTTPLARAAWETARHTPVNSWLGNIIMYAPTLWARMILMTHFF
SILLAQEQLEKALDCQIYGATYSIEPLDLPQIIQRLHGLSAFSLHSYSPGEINRVAS
CLRKLGVPPLRVWRHRARSVRAKLLSQGGRAATCGKYLFNWAVKTKLKLTPIPEASQ
LDLSGWFVAGYSGGDIYHSLSRARPRWFMWCLLLLSVGVGIYLLPNR).

The sequences of SEQ ID NOS: 84 to 123 are sequences consisting of 9 amino acid residues contained in a certain genome protein of the HCV pBRT703'X strain (mutant subclone of D89815), obtained from Professor Yoshiharu Matsuura, at the research institute for Microbial diseases at Osaka University. Furthermore, the sequences of SEQ ID NOS: 84 to 123 are sequences having superior binding to an HLA-A24 type molecule as predicted by a hypotheses obtained by the experimental design method explained in Embodiment 1. SEQ ID NOS: 84 to 123 are arranged in decreasing binding order. That is, SEQ ID NO: 84 is the sequence that is predicted to have the best binding. The full-length amino acid sequence of the certain genome protein of the HCV pBRT703'X strain (mutant subclone of D89815) is shown in SEQ ID NO: 186

(MSTNPKPQRKTKRNTNRRPQDVKFPGGGQIVGGVYLLPRRGPRLGVRATRKTSERS
QPRGRRQPIPKARHPEGRAWAQPGYPWPLYGNEGMGWAGWLLSPRGSRPSWGPTDPR
RRSRNLGKVIDTLTCGFADLMGYIPLVGAPLGGAARALAHGVRVLEDGVNYATGNLP
GCSFSIFLLALLSCLTIPASAYEVRNVSGIYHVTNDCSNSSIVYEAADVIMHAPGCV
PCVRENNSSRCWVALTPTLAARNASVPTTTLRRHVDLLVGTAAFCSAMYVGDLCGSV
FLISQLFTFSPRRHETVQDCNCSIYPGHVSGHRMAWDMMMNWSPTAALVVSQLLRIP
QAVMDMVAGAHWGVLAGLAYYSMVGNWAKVLIVMLLFAGVDGHTRVTGGVQGHVTST
LTSLFRPGASQKIQLVNTNGSWHINRTALNCNDSLKTGFLAALFYTHKFNASGCPER
MASCRSIDKFDQGWGPITYAQPDNSDQRPYCWHYAPRQCGIVPASQVCGPVYCFTPS
PVVVGTTDRFGAPTYNWGDNETDVLLLNNTRPPHGNWFGCTWMNSTGFTKTCGGPPC
NIRGVGNNTLTCPTDCFRKHPDATYTKCGSGPWLTPRCLVDYPYRLWHYPCTVNFTI
FKVRMYVGGVEHRLDAACNWTRGERCDLEDRDRAELSPLLLSTTEWQILPCSYTTLP
ALSTGLIHLHQNIVDIQYLYGIGSAVVSIAIKWEYVVLLFLLLADARVCACLWMMLL
IAQAEAALENLVVLNAASVAGAHGILPFFMFFCAAWYMKGRLVPGAAYAFYGVWPLL
LLLLALPPRAYAMDREMAASCGGGVFVGLALLTLSPYCKVFLARLIWWLQYFITKAE

AHLQVWVPPLNVRAGRDAIILLMCAVHPELIFDITKLLLSILGPLMVLQASLIRVPY

FVRAQGLIRACTLVRKAAGGHYVQMAFVKLAALTGTYVYDHLTPLQDWAHVGLRDLA

VAVEPVVFSAMETKVITWGADTAACGDIISGLPVSARRGKEILLGPADSFEGQGWRL

LAPITAYSQQTRGLLGCIITSLTGRDKNQVEGEVQVVSTATQSFLATCVNGACWTVF

HGAGSKTLAGPKGPITQMYTNVDQDLVGWPAPPGARSLTPCTCGSSDLYLVTRHADV

IPVRRRGDTRGSLLSPRPISYLKGSSGGPLLCPSGHVVGIFRAAVCTRGVAKAVDFI

PVESMETTMRSPVFTDNSTPPAVPQTFQVAHLHAPTGSGKSTKVPAAYAAQGYMVLV

LNPSVAATLGFGAYMSKAHGIDPNIRTGVRTITTGAPITYSTYGKFLADGGCSGGAY

DIIICDECHSTDSTSILGIGTVLDQAETAGARLVVLATATPPGSVTFPHPNIEEVAL

GNTGEIPFYGKAIPIEVIKGGRHLIFCHSKKKCDELAAKLSPLGLNAVAYYRGLDVS

VIPASGDVVVVATDALMTGFTGDFDSVIDCNTCVTQTVDFSLDPTFTIETTTVPQDA

VSRTQRRGRTGRGRRGIYRFVTPGERPSGMFDSSVLCECYDAGCAWYELTPAETSVR

LRAYLNTPGLPVCQDHLEFWESVFTGLTHIDAHFLSQTKQAGDNFPYLVAYQATVCA

RAKAPPPSWDQMWKCLIRLKPTLHGPTPLLYRLGAVQNEVTLTHPITKFIMACMSAD

LEVVTSTWVLVGGVLAALAAYCLTTGSVVIVGRIILSGRPAVIPDREVLYQEFDEME

ECASHLPYIEQGMQLAEQFKQKALGLLQTATKQAEAAAPVVESKWRALETFWAKHMW

NFISGIQYLAGLSTLPGNPAIASLMAFTASITSPLATQYTLLFNILGGWVAAQLAPP

SAASAFVGAGIAGAAVGSIGLGKVLVDILAGYGAGVAGALVAFKVMSGDMPSTEDLV

NLLPAILSPGALVVGVVCAAILRRHVGPGEGAVQWMNRLIAFASRGNHVSPTHYVPE

SDAAARVTQILSNLTITQLLKRLHQWINEDCSTPCSGSWLRDVWDWICTVLADFKTW

LQSKLLPRLPGVPFFSCQRGYKGVWRGDGIMYTTCPCGAQITGHVKNGSMRIVGPRT

CSNTWHGTFPINAYTTGPCTPSPAPNYSRALWRVAAEEYVEVTRVGDFHYVTGMTTD

NVKCPCQVPAPEFFTELDGVRLHRYAPACKPLLRDEVTFQVGLNQYTVGSQLPCEPE

PDVTVVTSMLTDPSHITAEAARRRLARGSPPSLAGSSASQLSAPSLKATCTTHHGAP

DTDLIEANLLWRQEMGGNITRVESENKIVILDSFEPLRAEEDEREVSAAAEILRKTR

KFPAAMPVWARPDYNPPLLESWKNPDYVPPVVHGCPLPPTKAPPIPPPRRKRTVVLT

ESTVSSALAELATKTFGGSGSSAVDSGTATGPPDQASAEGDAGSDAESYSSMPPLEG

EPGDPDLSDGSWSTVSEEASEDVVCCSMSYTWTGALITPCAAEESKLPINALSNPLL
RHHNMVYSTTSRSASLRQKKVTFDRMQVLDDHYRDVLKEMKAKASTVKAKLLSVEEA
CKLTPPHSAKSKFGYGAKDVRSLSSRAVNHIRSVWKDLLEDTDTPIQTTIMAKNEVF
CVQPEKGGRKPARLIVFPDLGVRVCEKMALYDVVSTLPQAVMGSSYGFQYSPKQRVE
FLVNTWKAKKCPMGFSYDTRCFDSTVTENDIRVEESIYQCCDLAPEARQAIRSLTER
LYIGGPMTNSKGQNCGYRRCRASGVLTTSCGNTLTCYLKAAAACRAAKLQDCTMLVC
GDDLVVICDSAGTQEDAASLRVFTEAMTRYSAPPGDPPQPEYDLELITSCSSNVSVA
HDASGKRVYYLTRDPTTPLARAAWETARHTPVNSWLGNIIMYAPTLWARMILMTHFF
SILLAQEQLEKALDCQIYGATYSIEPLDLPQIIQRLHGLSAFSLHSYSPGEINRVAS
CLRKLGVPPLRVWRHRARSVRAKLLSQGGRAATCGKYLFNWAVKTKLKLTPIPEASQ
LDLSGWFVAGYSGGDIYHSLSRARPRWFMWCLLLLSVGVGIYLLPNR).

The sequences of SEQ ID NOS: 124 to 183 are sequences consisting of 9 amino acid residues contained in a certain genome protein of the above-mentioned HCV pBRT703'X strain (mutant subclone of D89815). Furthermore, the sequences of SEQ ID NOS: 124 to 183 are sequences having superior binding to an HLA-A2 type molecule as predicted by a hypotheses obtained by the experimental design method explained in Embodiment 1. SEQ ID NOS: 124 to 183 are arranged in decreasing binding order. That is, SEQ ID NO: 124 is the sequence that is predicted to have the best binding.

Table 1 to Table 4 show amino acid sequences that had superior scores in the predicted results obtained using the above-mentioned prediction program, the predicted score, and the corresponding binding experiment data for the HCV D90208 strain, the D89815 strain, and the pBRT703'X strain (mutant subclone of D89815). All of the binding experiment data were obtained by artificially synthesizing 9 amino acid peptides by the above-mentioned synthetic method.

Said certain genome proteins of the HCV D90208 strain and the D89815 5 strain are registered in the GenBank, but the sequences consisting of 9 amino acid residues therein, which are the HLA-binding peptides, are not currently registered.

Furthermore, the pBRT703'X strain (mutant subclone of D89815) is a mutant strain that is similar to a substrain of HCV often seen in Japanese hepatitis C patients. In the present example, an HLA-binding peptide contained in a certain genome protein of the mutant strain similar to the substrain often seen in Japanese people has been found. This HLA-binding peptide can suitably be used for the development of a hepatitis C therapeutic drug for Japanese people.

Here, the amino acid sequences of the certain genome proteins of the above-mentioned HCV D90208 strain, D89815 strain, and pBRT703'X strain (mutant subclone of D89815) are different from each other in part, and it can be predicted that even an amino acid sequence formed by substitution of one or a few amino acid residues in the amino acid sequence will similarly show excellent HLA-binding properties as described above.

For example, the sixth peptide from the left of SEQ ID NO: 1 of the D90208 strain is F, but it is Y for the peptide of SEQ ID NO: 46 of the D89815 strain and the peptide of SEQ ID NO: 85 of the pBRT703'X strain (mutant subclone of D89815).

Furthermore, the second peptide from the left of SEQ ID NO: 17 of the D90208 strain is L, but it is F for the peptide of SEQ ID NO: 49 of the D89815 strain and the peptide of SEQ ID NO: 98 of the pBRT703'X strain (mutant subclone of D89815).

Moreover, the fifth peptide from the left of SEQ ID NO: 3 of the D90208 strain is V, but it is A for the peptide of SEQ ID NO: 71 of the D89815 strain and the peptide of SEQ ID NO: 110 of the pBRT703'X strain (mutant subclone of D89815).

Furthermore, the seventh peptide from the left of SEQ ID NO: 2 of the D90206 strain is D, but it is E for the peptide of SEQ ID NO: 45 of the D89815 strain and the peptide of SEQ ID NO: 84 of the pBRT703'X strain (mutant subclone of D89815).

Moreover, the seventh peptide from the left of SEQ ID NO: 40 of the D90208 strain is C, but it is G for the peptide of SEQ ID NO: 107 of the pBRT703'X strain (mutant subclone of D89815).

Furthermore, the fifth peptide from the left of SEQ ID NO: 43 of the D90208 strain is E, but it is D for the peptide of SEQ ID NO: 59 of the D89815 strain and the peptide of SEQ ID NO: 87 of the pBRT703'X strain (mutant subclone of D89815), and the eighth peptide from the left of SEQ ID NO: 43 of the D90208 strain is V, but it is T for the peptide of SEQ ID NO: 59 of the D89815 strain and the peptide of SEQ ID NO: 87 of the pBRT703'X strain (mutant subclone of D89815).

Moreover, the seventh peptide from the left of SEQ ID NO: 44 of the D90208 strain is I, but it is V for the peptide of SEQ ID NO: 62 of the D89815 strain and the peptide of SEQ ID NO: 93 of the pBRT703'X strain (mutant subclone of D89815), and the ninth peptide from the left of SEQ ID NO: 44 of the D90208 strain is V, but it is F for the peptide of SEQ ID NO: 62 of the D89815 strain and the peptide of SEQ ID NO: 93 of the pBRT703'X strain.

Among the peptide sequences formed by substitution of one or two amino acid residues with each other, for example, the second peptide from the left of SEQ ID NO: 17 of the D90208 strain is L, but it is F for the peptide of SEQ ID NO: 49 of the D89815 strain and the peptide of SEQ ID NO: 98 of the pBRT703'X strain (mutant subclone of D89815), and the binding experimental value for the peptide of SEQ ID NO: 17 of the D90208 strain is 6.98038 whereas it is 7.7344 for the peptide of SEQ ID NO: 49 of the D89815 strain and the peptide of SEQ ID NO: 98 of the pBRT703'X strain (mutant subclone of D89815), thus confirming that they all show good binding properties.

Furthermore, among the peptide sequences formed by substitution of one or two amino acid residues with each other, for example, the seventh peptide from the left of SEQ ID NO: 40 of the D90208 strain is C, but it is G for the peptide of SEQ ID NO: 107 of the pBRT703'X strain (mutant subclone of D89815), and the binding experimental value for the peptide of SEQ ID NO: 40 of the D90208 strain is 6.97507 whereas it is 6.37373 for the peptide of SEQ ID NO: 107 of the pBRT703'X strain (mutant subclone of D89815), thus confirming that they all show good binding properties.

Moreover, among the peptide sequences formed by substitution of one or two amino acid residues with each other, for example, the fifth peptide from the left of SEQ ID NO: 3 of the D90208 strain is V, but it is A for the peptide of SEQ ID NO: 71 of the D89815 strain and the peptide of SEQ ID NO: 110 of the pBRT703'X strain (mutant subclone of D89815), and the binding experimental value for the peptide of SEQ ID NO: 3 of the D90208 strain is 6.14848 whereas it is 6.75756 for the peptide of SEQ ID NO: 71 of the D89815 strain and the peptide of SEQ ID NO: 110 of the pBRT703'X strain (mutant subclone of D89815), thus confirming that they all show good binding properties.

Furthermore, among the peptide sequences formed by substitution of one or two amino acid residues with each other, for example, the seventh peptide from the left of SEQ ID NO: 2 of the D90208 strain is D, but it is E for the peptide of SEQ ID NO: 45 of the D89815 strain and the peptide of SEQ ID NO: 84 of the pBRT703'X strain (mutant subclone of D89815), and the binding experimental value for the peptide of SEQ ID NO: 2 of the D90208 strain is 5.32417 whereas it is 5.00343 for the peptide of SEQ ID NO: 45 of the D89815 strain and the peptide of SEQ ID NO: 84 of the pBRT703'X strain (mutant subclone of D89815), thus confirming that they all show good binding properties.

Moreover, among the peptide sequences formed by substitution of one or two amino acid residues with each other, for example, the amino acid sequence is offset sideways by one between the peptide of SEQ ID NO: 90 and the peptide of SEQ ID NO: 112 of the pBRT703'X strain (mutant subclone of D89815), and the binding experimental value for the peptide of SEQ ID NO: 90 is 6.51874 whereas the binding experimental value for the peptide of SEQ ID NO: 112 is 6.63423, thus confirming that they all show good binding properties.

Furthermore, among the peptide sequences formed by substitution of one or two amino acid residues with each other, for example, the amino acid sequence is offset sideways by one between the peptide of SEQ ID NO: 13 of the D90208 strain and the peptide of SEQ ID NO: 60 of the D89815 strain and the peptide of SEQ ID NO: 18 of the D90208 strain and the peptide of SEQ ID NO: 64 of the D89815 strain, and the binding experimental value for the peptides of SEQ ID NOS: 13 and 60 is 7.89519 whereas the binding experimental value for the peptides of SEQ ID NOS: 18 and 64 is 5.9169, thus confirming that they all show good binding properties.

It can therefore be predicted that both the peptide sequences formed by substitution of one or two amino acid residues with each other will show excellent binding to an HLA-A24 type molecule. In conclusion, even an amino acid sequence formed by deletion, substitution, or addition of one or a few amino acid residues of an amino acid sequence that has excellent properties in binding to an HLA-A type molecule shown by SEQ ID NOS: 1 to 183 can be predicted to similarly show excellent HLA-binding properties.

From another viewpoint, even an amino acid sequence formed by deletion, substitution, or addition of one or a few amino acid residues of an amino acid sequence that has excellent properties in binding to an HLA-A type molecule as predicted by the hypothesis obtained by the experimental design method explained in Embodiment 1 similarly can be said to show excellent HLA-binding properties. The amino acid residues that are substituted are preferably amino acid residues that have similar properties to each other, such as the two being hydrophobic amino acid residues.

The present invention is explained above by reference to Examples. These Examples are only illustrated as examples, and a person skilled in the art will understand that various modification examples are possible, and such modification examples are included in the scope of the present invention.

For example, in the above-mentioned Examples, HCV D90208 strain, D89815 strain, and pBRT703'X strain (mutant subclone of D89815) were used, but another HCV strain may be used. In this case, in accordance with the prediction program employed in the present invention, the HLA binding can be predicted with high precision.

## Claims

1. An HLA-binding peptide binding to an HLA-A type molecule, said HLA-binding peptide comprising
at least one type of amino acid sequence selected from the group consisting of SEQ ID NOS: 1 to 183, and
not less than 8 and not more than 11 amino acid residues.

2. The HLA-binding peptide as set forth in Claim 1, comprising at least one type of amino acid sequence selected from the group consisting of SEQ ID NOS: 1, 2, 3, 5, 8, 12, 13, 14, 16, 17, 18, 19, 22, 23, 25, 27, 34, 37, 38, 40, 42, 45, 48, 49, 52, 53, 54, 55, 56, 58, 59, 60, 61, 62, 63, 64, 65, 67, 71, 72, 74, 75, 76, 84, 86, 87, 90, 91, 92, 93, 94, 96, 97, 98, 100, 101, 102, 104, 106, 107, 108, 109, 110, 112, 123, 124, 126, 127, 131, 132, 133, 134, 135, 136, 137, 139, 141, 142, 146, 147, 149, 150, 152, 162, 170, 173, 176, 177, and 179.

3. An HLA-binding peptide binding to an HLA-A type molecule, said HLA-binding peptide comprising
an amino acid sequence formed by deletion, substitution, or addition of one or two amino acid residues of said amino acid sequence contained in the HLA-binding peptide as set forth in Claim 1 or 2, and
not less than 8 and not more than 11 amino acid residues.

4. The HLA-binding peptide as set forth in any one of Claims 1 to 3, wherein said human HLA-binding peptide binds to a human HLA-A24 type molecule.

5. The HLA-binding peptide as set forth in any one of Claims 1 to 3, wherein said human HLA-binding peptide binds to a human HLA-A2 type molecule.

6. A DNA segment comprising a DNA sequence coding for the HLA-binding peptide as set forth in any one of Claims 1 to 5.

7. A recombinant vector comprising a DNA sequence coding for the HLA-binding peptide as set forth in any one of Claims 1 to 5.

8. An HLA-binding peptide precursor changing into the HLA-binding peptide as set forth in any one of Claims 1 to 5 within a mammalian body.
